# EUROPEAN PATENT APPLICATION

(11) **EP 1 125 921 A1**
(43) Date of publication of application: **22.08.2001**
(21) Application number: 99947956.1
(22) Date of filing: 19.10.1999
(51) Int. Cl.: C07C 403/22, C07C 403/08

(54) **PROCESS FOR THE PREPARATION OF RETINOL AND INTERMEDIATES THEREFOR**

(30) Priority: 26.10.1998 JP 30385198
(71) Applicant: SUMITOMO CHEMICAL COMPANY LIMITED, Osaka-shi, Osaka 541-0041 (JP)
(72) Inventor: TAKAHASHI, Toshiya, Ibaraki-shi, Osaka 567-0826 (JP); FURUTANI, Atsushi, Takatsuki-shi, Osaka 569-0857 (JP); SEKO, Shinzo, Toyonaka-shi, Osaka 560-0003 (JP)
(74) Representative: Henkel, Feiler, Hänzel
(86) International application number: JP9905748
(87) International publication number: WO0024713

(57) **Abstract**

The reaction of a halogenated sulfone derivative of general formula (1): 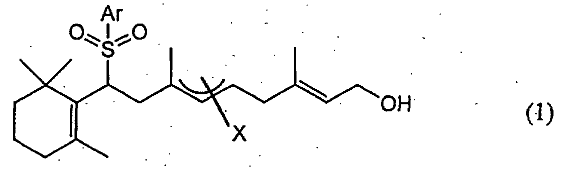 wherein Ar is optionally substituted aryl and X is halogen, with a base can attain easy production of retinol useful in the fields of drugs, feed derivatives and food derivatives. The intermediate in this production can easily be obtained by reaction of a diol compound of general formula (2): 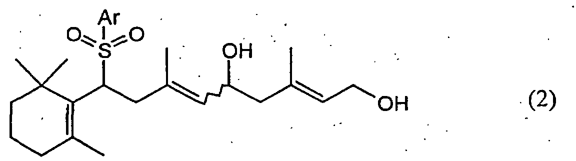 wherein Ar is as defined above, which can be derived from relatively inexpensive starting materials, with a Group IV transition metal halide without protecting the primary hydroxyl group.

## Description

### Technical Field

The present invention relates to a production process for retinol which is useful in the fields of drugs, feed additives and food additives, and to intermediates therein.

### Background Art

As the production process for retinol derivatives, there have so far been reported a process of increasing the number of carbon atoms on the side chain of a C₁₃ carbonyl compound (β-ionone) as the key intermediate (*e.g*., Pure & Appl. Chem. Vol. 66, p. 1509-1518 (1994)) and a process of coupling a C₁₀ sulfonyl compound (cyclogeranyl sulfone) as the key intermediate to a C₁₀ aldehyde derivative (*e*.*g*., JP-B 5-61265). These processes, however, cannot always be considered excellent on an industrial point of view because of their drawbacks, for example, the synthesis of β-ionone as the key intermediate in the former process needs a multi-step process and this compound becomes an expensive starting material, and the synthesis of a C₁₀ aldehyde derivative in the latter process needs an oxidizing agent difficult to obtain (JP-A 7-17555). Under these circumstances, the present inventors have found that diol compounds of general formula (2) as shown below can be obtained by coupling reaction of C₁₀ sulfonyl compounds to C₁₀ allyl halide derivatives, which can be derived using inexpensive starting materials. In the subsequent step of halogenating the compounds containing both the primary and the secondary hydroxyl groups in their molecules, such as diol compounds (2), however, it is usually necessary to protect the primary hydroxyl groups for the purpose of halogenating only the secondary hydroxyl groups, which needs a multi-step process.

### Disclosure of Invention

In view of these drawbacks, the present inventors have extensively studied to develop a process for conversion of diol compound (2), which can easily be synthesized from relatively inexpensive starting materials, linalool or geraniol, into retinol at reduced steps without taking a route through C₁₃ β-ionone or C₁₀ aldehyde derivatives. As a result, they have found that the use of a particular halogenating agent makes possible selective halogenation of only the secondary hydroxyl group of diol compound (2) without protecting the coexisting primary hydroxyl group, resulting in the present invention.

That is, the present invention provides a halogenated sulfone derivative of general formula (1): 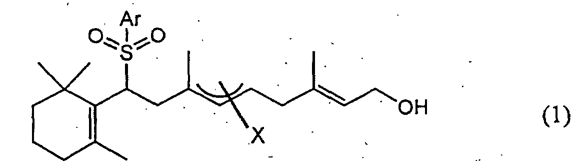 wherein Ar is optionally substituted aryl and X is halogen, which can be obtained by reaction of a diol compound of general formula (2): 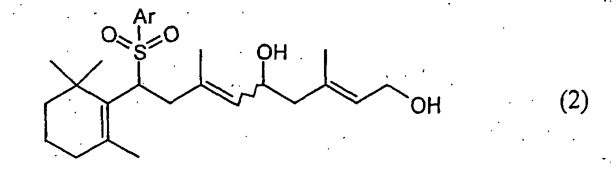 wherein Ar is as defined above, with a Group IV transition metal halide; and a production process for retinol by reaction of such halogenated. sulfone derivative with a base to eliminate the substituent groups.

### Mode for Carrying Out the Invention

The halogenated sulfone derivative of general formula (1) can be obtained by reaction of a diol compound of general formula (2) with a Group IV transition metal halide. This reaction makes possible selective halogenation of only the secondary hydroxyl group without protecting the primary hydroxyl group.

In the halogenated sulfone derivative (1) and diol compound (2) of the present invention, substituent group Ar refers to optionally substituted aryl, and such aryl may include phenyl and naphthyl optionally substituted with C₁-C₅ alkyl, C₁-C₅ alkoxy, halogen, nitro, or other groups. Specific examples are phenyl, naphthyl, o-tolyl, m-tolyl, p-tolyl, o-methoxyphenyl, m-methoxyphenyl, p-methoxyphenyl, o-chlorophenyl, m-chlorophenyl, p-chlorophenyl, o-bromophenyl, m-bromophenyl, p-bromophenyl, o-iodophenyl, m-iodophenyl, p-iodophenyl, o-fluorophenyl, m-fluorophenyl, p-fluorophenyl, o-nitrophenyl, m-nitrophenyl, p-nitrophenyl, and the like.

In the halogenated sulfone derivative (1), substituent group X refers to halogen, specific examples of which are chlorine, bromine, and iodine.

The Group IV transition metal halide used in the above reaction may include titanium tetrachloride, titanium tetrabromide, titanium tetraiodide, titanium dichlorodiisopropoxide, titanium chlorotriisopropoxide, zirconium tetrachloride, zirconium tetrabromide, and hafnium tetrachloride. More preferably, a chloride of titanium is used, with titanium tetrachloride being particularly preferred.

The amount of Group IV transition metal halide to be used is usually in the range of 0.1 to 5 moles, preferably 0.2 to 3 moles, relative to 1 mole of diol compound (2).

In the above reaction, an organic solvent is usually used. The organic solvent may include ether solvents such as diethyl ether, tetrahydrofuran, dimethoxyethane, anisole and 1,4-dioxane; halogenated solvents such as chloroform, dichloromethane, 1,2-dichloroethane, monochlorobenzene and o-dichlorobenzene; and hydrocarbon solvents such as n-hexane, cyclohexane, n-pentane, n-heptane, toluene and xylene. Preferably, an ether solvent is used. These solvents may be used as a mixed solvent. When an organic solvent other than ether solvents is used, an ether compound may preferably be used at the same time. Such ether compound may include diethyl ether, tetrahydrofuran, dimethoxyethane, anisole, and 1,4-dioxane. The amount for its use is enough even in the range of 1 to 10 moles, relative to 1 mole of diol compound (2).

The reaction temperature is usually in the range of -20°C to the. boiling point of a solvent used, preferably 20°C to 60°C. The reaction time may vary with the kind of metal halide used in the reaction and the reaction temperature; however, it is usually in the range of about 1 to 24 hours.

After the reaction, the reaction mixture is subjected to an ordinary: post-treatment, which affords a halogenated sulfone derivative of general formula (1).

The starting material, diol compound (2), may be any of E- or Z-type geometrical isomer, its mixture, or a mixture of diastereomers, or a racemate or an optical active form.

The diol compound (2), which is the starting compound of the present invention, can easily be synthesized from linalool or geraniol available at a relatively low cost, by a route as shown below in scheme 1. The synthesis process for cyclogeranyl sulfone (3) is described in Chem., Lett., 479 (1975) and the synthesis process for diol compound (2) is described in EP-900785A2.

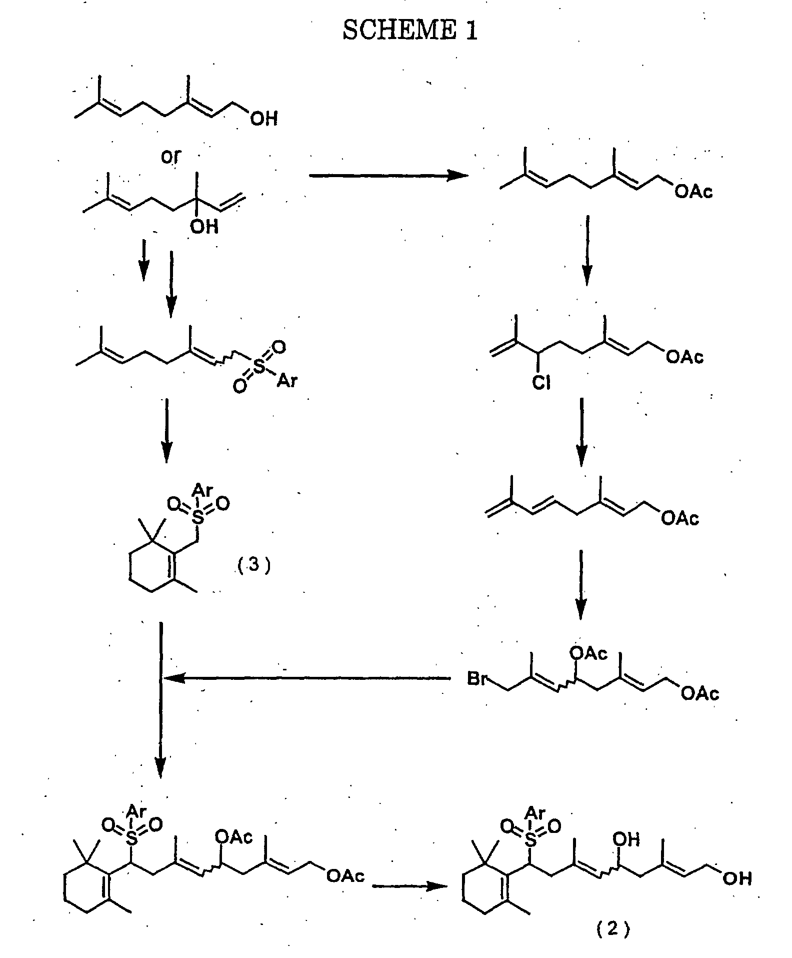

The halogenated sulfone derivative (1) of the present invention can easily be converted into retinol by a reaction with a base.

The base used in this reaction may include alkali metal compounds and alkaline earth metal compounds, such as alkali metal hydroxides, alkaline earth metal hydroxides, alkali metal alkoxides and alkaline earth metal alkoxides. Specific examples are sodium hydroxide, potassium hydroxide, magnesium hydroxide, sodium methoxide, potassium methoxide, sodium t-butoxide, potassium t-butoxide, calcium methoxide, and magnesium methoxide. The amount for its use is usually in the range of about 1 to 40 moles, relative to 1 mole of the halogenated sulfone derivative (1).

In the above reaction, an organic solvent is usually used. The organic solvent may include hydrocarbon solvents such as n-hexane, cyclohexane, n-pentane, n-heptane, toluene and xylene; ether solvents such as diethyl ether, tetrahydrofuran, dimethoxyethane and anisole; alcohol solvents such as methanol, ethanol and isopropanol; and aprotic solvents such as N,N-dimethylformamide, dimethylsulfoxide, N,N-dimethylacetamide and hexamethylphosphoric acid triamide. These solvents may be used as a mixed solvent.

The reaction temperature is usually in the range of 0°C to the boiling point of a solvent used, preferably 15°C to 50°C. The reaction time may vary with the kind of base or solvent used in the. reaction and is usually in the range of about 1 to 24 hours.

After the reaction, the reaction mixture is subjected to an ordinary post-treatment, which affords retinol. If necessary, it may be purified by recrystallization, chromatography, or any other technique.

The retinol can be converted into retinyl acetate by introduction of protective groups for the hydroxyl groups, for example, by their acetylation, according to ordinary methods.

### Examples

The present invention will hereinafter be further illustrated by some examples; however, the present invention is not limited to these examples.

### Example 1

In a dried 4-necked flask, 0.46 g (1.0 mmol) of 1,5-dihydroxy-3,7-dimethyl-9-(2,6,6-trimethylcyclohexen-1-yl)-9-(4-methylphenylsulfonyl)-nona-2,6-diene (hereinafter referred to as compound (a)) was dissolved in 10 ml of dimethoxyethane (DME) under a nitrogen gas, to which 0.095 g (0.50 mmol) of titanium tetrachloride was added dropwise at room temperature, and the mixture was stirred at the same temperature for 12 hours and then at 50°C for 4 hours. The reaction mixture was quenched with a 1% aqueous sodium hydroxide solution and extracted with ethyl acetate. The resulting organic layer was dried over anhydrous magnesium sulfate and then evaporated to remove the solvent, which afforded a crude product (mixture (b) of eight compounds including 1,3-allyl isomers at the chlorine-bonding position, cis-trans isomers on the olefinic linkages, and diastereomers) as a pale brown oil in 95% yield. A major product among them was 1-hydroxy-5-chloro-3,7-dimethyl-9-(2,6,6-trimethylcylcohexen-1-yl)-9-(4-methylphenylsulhonyl)-nona-2,6-diene (hereinafter referred to as compound (c)).

### Physical Properties of Compound (c):

¹H-NMR (CDCl₃) δ(ppm): 0.82-2.64 (m, 27H), 3.03-3.17 (m, 1H), 3.91 (m, 1H), 4.13 (m, 2H), 4.55-4.78 (m, 1H), 5.39 (d, J = 8 Hz, 1H), 5.53 (m, 1H), 7.10-7.41 (m, 2H), 7.65-7.85 (m, 2H).
HR-MS: Calculated (for C₂₇H₃₈ClO₂S): 461.2203
Observed: 461.2236 (M-OH)⁺.

### Examples 2 to 6

The same reaction was carried out in the same manner as described in Example 1, except that the reaction conditions as shown below in Table 1 were employed, and the results were obtained as shown in Table 1.

**TABLE 1**

| Example | Metal halide | Molar ratio¹⁾ | Solvent | Time (h) | Yield (%) |
|---|---|---|---|---|---|
| 2 | TiCl₄²⁾ | 1.2 | THF | 1 | 94 |
| 3 | TiCl₄²⁾ | 1.2 | DME | 1 | 99 |
| 4 | TiCl₄²⁾ | 0.25 | DME | 4 | 99 |
| 5 | TiCl₄²⁾ | 0.3 | toluene³⁾ | 5 | 90 |
| 6 | TiCl₂(OⁱPr)²⁾ | 2.0 | DME | 10 | 96 |

| | | | | | |
|---|---|---|---|---|---|
| Reaction temperature: 50°C, THF: tetrahydrofuran, DME: dimethoxyethane ¹⁾ Molar ratio of metal halide to compound (a), | | | | | |
| ²⁾ 1M toluene solution, | | | | | |
| ³⁾ DME was added at a molar ratio of 5 to compound (a). | | | | | |

### Example 7

First, 676 mg (1.41 mmol) of crude product (b) obtained in accordance with Example 1 was dissolved in 11 ml of cyclohexane, to which 988 mg (14.1 mmol) of potassium methoxide was added, and the mixture was stirred at 40°C for 2 hours. The reaction mixture was quenched with a saturated aqueous ammonium chloride solution and extracted with ethyl acetate. The resulting organic layer was washed again with saturated aqueous ammonium chloride solution, dried over anhydrous magnesium sulfate, and then evaporated to remove the solvent, which afforded crude product (d) as a red oil. The resulting crude product was found to be retinol by NMR analysis. The resulting crude product (d) was dissolved in 8 ml of toluene, to which 112 mg (1.41 mmol) of pyridine and 17 mg (0.141 mmol) of dimethylaminopyridine were added and 158 mg (1.55 mmol) of acetic anhydride was then slowly added at 5°C, and the mixture was stirred at room temperature for 18 hours. The reaction mixture was quenched with a 5% hydrochloric acid and extracted with toluene. The resulting organic layer was sequentially washed with a saturated aqueous sodium bicarbonate solution and a saturated sodium chloride solution, dried over anhydrous magnesium sulfate, and then evaporated to remove the solvent, which afforded a crude product as a yellow oil. The analysis of the resulting crude product by liquid chromatography revealed that the total yield of retinyl acetate (e) (from compound (a)) was 74%.

### Example 8

First, 676 mg (1.41 mmol) of crude product (b) obtained in accordance with Example 1 and 45 mg of methanol (1.41 mmol) were dissolved in 12 ml of toluene, to which 79 mg (14.1 mmol) of potassium hydroxide and 23 mg (0.071 mmol) of tetrabutylammonium bromide were added, and the mixture was stirred at 40°C for 12 hours. The reaction mixture was quenched with a saturated aqueous ammonium chloride solution and extracted with ethyl acetate. The resulting organic layer was washed again with a saturated aqueous ammonium chloride solution, dried over anhydrous magnesium sulfate, and then evaporated to remove the solvent, which afforded crude product (d) as a red oil. The resulting crude product was found to be retinol by NMR analysis. The resulting crude product (d) was dissolved in 8 ml of toluene, to which 112 mg (1.41 mmol) of pyridine and 17 mg (0.141 mmol) of dimethylaminopyridine were added and 158 mg (1.55 mmol) of acetic anhydride was then slowly added at 5°C, and the mixture was stirred at room temperature for 18 hours. The reaction mixture was quenched with a 5% hydrochloric acid and extracted with toluene. The resulting organic layer was sequentially washed with a saturated aqueous sodium bicarbonate solution and a saturated sodium chloride solution, dried over anhydrous magnesium sulfate, and then evaporated to remove the solvent, which afforded a crude product as a yellow oil. The analysis of the resulting crude product by liquid chromatography revealed that the total yield of retinyl acetate (e) (from compound (a)) was 56%.

### Reference Example 1

First, 40 g (0.204 mol) of geranyl acetate was dissolved in 100 ml of n-hexane, to which 17.1 g (0.071 mol) of trichloroisocyanuric acid was slowly' added, and the mixture was kept at -10°C to 0°C for 6 hours. After the reaction, excess trichloroisocyanuric acid and a by-product, isocyanuric acid were removed by filtration. The filtrate was sequentially washed with an aqueous sodium bicarbonate solution and water, dried over anhydrous magnesium sulfate, and then evaporated to remove the solvent, which afforded a crude product. The resulting crude product was purified by silica gel column chromatography, which afforded 6-chloro-3,7-dimethyl-2,7-octadien-1-acetate (hereinafter referred to as compound (f)) as a pale yellow oil in 86% yield.
¹H-NMR (CDCl₃) δ(ppm): 1.71 (s, 3H), 1.81 (s, 3H), 1.90-2.22 (m, 4H), 2.05 (s, 3H), 4.34 (t, J = 7 Hz, 1H), 4.59 (d, J = 7 Hz, 2H), 4.90 (s, 1H), 5.01 (s, 1H), 5.37 (t, J = 7 Hz, 1H).

### Reference Example 2

To a dried 4-necked flask, 6.8 g (0.17 mol) of sodium hydroxide in fine powder, 2.2 g (8.5 mmol) of triphenylphosphine, 1.4 g (5.1 mmol) of tetra-n-butylammonium chloride, 0.62 g (1.7 mmol) of allyl palladium chloride dimer, and 100 ml of THF were added under a nitrogen gas. Then, 150 ml of a THF solution of 40 g (0.17 mol) of compound (f) was added thereto dropwise under stirring at room temperature over 1 hour. After stirring at room temperature for 3 days, the reaction mixture was quenched with water and extracted with ether. The resulting organic layer was washed with a saturated sodium chloride solution, dried over anhydrous magnesium sulfate, and then evaporated to remove the solvent, which afforded a crude product. The resulting crude product was purified by silica gel chromatography, which afforded 3,7-dimethyl-2,5,7-octatriene-1-acetate (hereinafter referred to as compound (g)) in 65% yield.
¹H-NMR (CDCl₃) δ(ppm): 1.70 (s, 3H), 1.85 (s, 3H), 2.08 (s, 3H), 2.81 (d, J = 7 Hz, 2H), 4.58 (d, J = 7 Hz, 2H), 4.90 (s, 2H), 5.37 (t, J = 7 Hz, 1H), 5.61 (td, J = 16, 7 Hz, 1H), 6.16 (d, J = 16 Hz, 1H).

### Reference Example 3

First, 20.1 g (0.1 mol) of compound (g) was dissolved in 100 ml of acetic acid, to which 18.3 g (0.1 mol) of N-bromosuccinimide was slowly added at room temperature. After 10 to 15 minutes at room temperature, the reaction solution became uniform, which was stirred for 2 hours, then quenched with water and extracted with toluene. The resulting organic layer was dried over anhydrous magnesium sulfate and then evaporated to remove the solvent, which afforded an about 1 : 1 mixture of 8-bromo-3,7-dimethyl-2,6-octadiene-1,5-diacetate (hereinafter referred to as compound (h); a mixture of EZ isomers) and 8-bromo-3,7-dimethyl-2,5-octadiene-1,7-diacetate (hereinafter referred to as compound (i); a mixture of EZ isomers) in 95% yield. The resulting mixture was separated and purified by silica gel chromatography, which afforded compounds (h) and (i) each as a pale yellow oil.

### Physical Properties of Compound (h):

¹H-NMR (CDCl₃) δ (ppm): 1.77 (s, 3H), 1.82 (s, 3H); 1.98 (s, 3H), 2.02 (s, 3H), 2.19 (m, 2H), 3.89 (s, 2H), 4.55 (d, J = 7 Hz, 2H), 5.37 (t, J =7 Hz, 1H), 5.48-5.62 (m, 2H).

### Physical Properties of Compound (i):

¹H-NMR (CDCl₃) δ (ppm): 1.65 (s, 3H), 1.68 (s, 3H), 2.05 (s, 3H), 2.06 (s, 3H), 2.78 (d, J = 6 Hz, 2H), 3.67 (d, J = 11 Hz, 1H), 3.82 (d, J = 11 Hz, 1H), 4.57 (d, J = 7 Hz, 2H), 5.35 (t, J = 7 Hz, 1H), 5.61-5.77 (m, 2H).

### Reference Example 4

First, 387 mg (1.99 mmol) of compound (g) was dissolved in 5 ml of acetonitrile, to which a solution of 400 mg (2.50 mmol) of bromine dissolved in 5 ml of water and 1 ml of acetonitrile was added dropwise at room temperature, and the mixture was stirred for 8 hours. The reaction mixture was quenched with water and extracted with ether. The resulting organic 'layer was washed with an aqueous sodium chloride solution, dried over anhydrous magnesium sulfate, and then evaporated to remove the solvent, which afforded an about 3 : 7 mixture of 8-bromo-5-hydroxy-3,7-dimethyl-2,6-octadiene-1-acetate (hereinafter referred to as compound (j); a mixture of EZ isomers) and 8-bromo-7-hydroxy-3,7-dimethyl-2,5-octadiene-1-acetate (hereinafter referred to as compound (k); a mixture of EZ isomers) in 98% yield. The resulting mixture was separated and purified by silica gel chromatography, which afforded compounds (j) and (k) each as a pale yellow oil.

### Physical Properties of Compound (j):

¹H-NMR (CDCl₃) δ (ppm): 1.76 (s, 3H), 1.82 (s, 3H), 2.06 (s, 3H), 2.20-2.30 (m, 1H), 2.67 (br, 1H), 3.95 (s, 2H), 4.41-4.53 (m, 1H), 4.59 (d, J = 7 Hz, 2H), 5.42 (t, J = 7 Hz, 1H), 5.58 (d, J = 9 Hz, 1H).

### Physical Properties of Compound (k):

¹H-NMR (CDCl₃) δ (ppm): 1.43 (s, 3H), 1.70 (s, 3H), 2.06 (s, 3H), 2.34 (br, 1H), 2.78 (d; J = 7 Hz, 2H), 3.47 (s, 2H), 4.59 (d, J = 7 Hz, 2H), 5.38 (t, J = 7 Hz, 1H), 5.57 (d, J = 7 Hz, 1H), 5.71-5.80 (m, 1H).

### Reference Example 5

First, 41 mg (0.14 mmol) of the 3 : 7 mixture of compounds (j) and (k) was dissolved in 0.5 ml of toluene, to which a mixture of 1 mg (0.007 mmol) of concentrated sulfuric acid and 15 mg (0.14 mmol) of acetic anhydride was added under stirring at 0°C, and the mixture was stirred at 25°C for 10 hours. The reaction mixture was quenched with water and extracted with n-hexane. The resulting organic layer was sequentially washed with a 5% aqueous sodium bicarbonate solution and a saturated sodium chloride solution, dried over anhydrous magnesium sulfate, and then evaporated to remove the solvent, which afforded compound (h) (a mixture of EZ isomers) as a pale yellow oil in 93% yield.

### Reference Example 6

First, 0.30 g (1.0 mmol) of β-cylcogeranyl-p-tolyl sulfone (hereinafter referred to as compound (1)) and 0.168 g (1.5 mmol) of potassium t-butoxide were dissolved in 8 ml of N,N-dimethylformamide (DMF), and the solution was cooled to -60°C. After stirring at the same temperature for 30 minutes, 3 ml of a DMF solution of 0.174g (0.5 mmol) of compound (h) was added dropwise, and the mixture was stirred at the same temperature for 2 hours. The reaction mixture was quenched with a saturated ammonium chloride solution and extracted with ethyl acetate. The resulting organic layer was washed with a saturated sodium chloride solution, dried over anhydrous magnesium sulfate, and then evaporated to remove the solvent, which afforded a mixture of three ingredients containing 1,5-acetoxy-3,7-dimethyl-9-(2,6,6-trimethylcyclohexen-1-yl)-9-(4-methylphenylsulfonyl)-2,6-nonadiene (hereinafter referred to as compound (m); a mixture of EZ isomers and diastereomers) and compounds which had been hydrolyzed on one of the acetoxy groups (a mixture of EZ isomers and diastereomers) as a yellow oil. The analysis of the resulting crude product by liquid chromatography revealed that the total yield of three ingredients was 96%.

### Physical Properties of Compound (m):

¹H-NMR (CDCl₃) δ (ppm): 0.73-1.04 (m, 6H), 1.38-1.70 (m, 6H), 1.39 (s, 3H), 1.70 (s, 3H), 2.00 (s, 3H), 2.01-2.31 (m, 2H), 2.01 (s, 3H), 2.03 (s, 3H), 2.44 (s, 3H), 2.66-2.95 (m, 2H), 3.82-3.86 (m, 1H), 4.53 (d, J = 7 Hz, 2H), 5.08-5.21 (m, 1H), 5.34 (br, 1H), 5.56 (br, 1H), 7.33 (d, J = 8 Hz, 2H), 7.76 (d, J = 8 Hz, 2H).
¹³C-NMR (CDCl₃) δ (ppm): 15.1, 16.0, 16.1, 16.6, 18.8, 20.8, 20.9, 21.4, 28.2, 29.0, 35.5, 40.5, 40.8, 44.6, 60.8, 65.3, 65.5, 65.7, 68.3, 68.5, 68.8, 121.9, 127.1, 128.3, 129.4, 130.5, 130.6, 136.2, 137.1, 137.6, 137.7, 138.4, 144.0, 169.8, 170.0, 170.7.

### Reference Example 7

First, 0.20 g (0.37 mmol) of compound (m) was dissolved in 5 ml of methanol, to which 0.11 g (0.74 mmol) of 27% aqueous sodium hydroxide solution was added under stirring, and the mixture was stirred at 25°C for 4 hours. The reaction mixture was quenched with a saturated aqueous ammonium chloride solution and extracted with ethyl acetate. The resulting organic layer was washed with a saturated sodium chloride solution, dried over anhydrous magnesium sulfate, and then evaporated to remove the solvent, which afforded 1,5-dihydroxy-3,7-dimethyl-9-(2,6,6-trimethylcyclohexen-1-yl)-9-(4-methylphenylsulfonyl)-2,6-nonadiene (compound (a); a mixture of EZ isomers and diastereomers) as a pale yellow oil in 95% yield.
¹H-NMR (CDCl₃) δ (ppm): 0.83-1.03 (m, 6H), 1.33-1.61 (m, 2H), 1.38 (s, 3H), 1.43 (s, 3H), 1.70 (s, 3H), 1.90-2.18 (m, 4H), 2.44 (s, 3H), 2.52-2.62 (m, 1H), 2.80-2.95 (br, 1H), 2.95-3.13 (m, 1H), 3.77-3.84 (m, 1H), 3.90 (t, J = 7 Hz, 1H), 4.03 (d, J = 7 Hz, 2H), 5.33-5.36 (m, 1H), 5.48-5.52 (t, J = 7 Hz, 1H), 7.30 (d, J = 8 Hz, 2H), 7.74 (d, J = 8 Hz, 2H).

The compounds used in the above examples and reference examples or the structures of the compounds obtained therein are shown below, in which "Ts" means p-tolylsulfonyl.

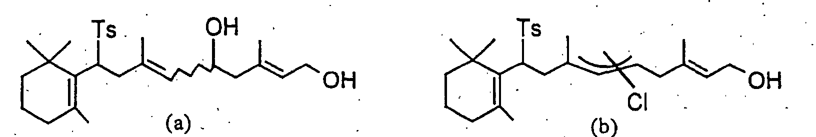

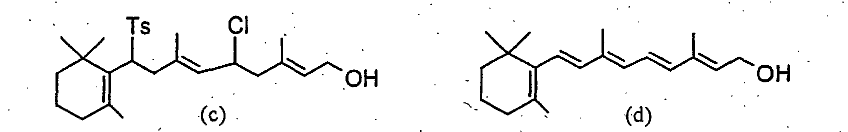

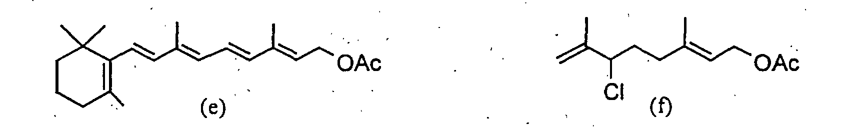

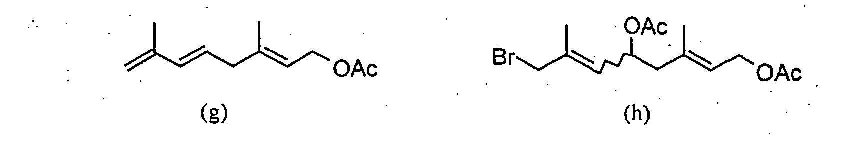

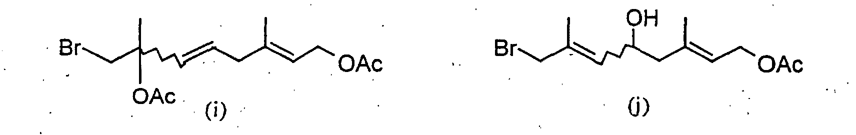

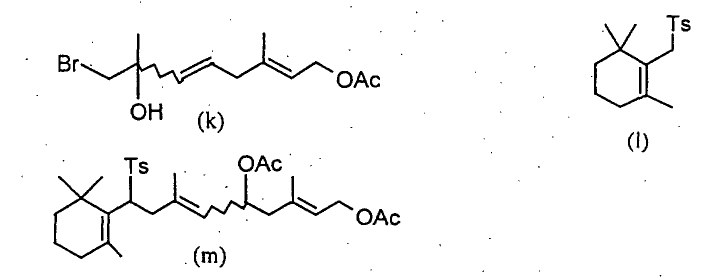

### Industrial Applicability

According to the present invention, direct and selective halogenation of the secondary hydroxyl group in the diol compound (2) can be achieved without protecting the primary hydroxyl group. The resulting halogenated sulfone derivative (1) can easily be converted into retinol by base treatment. The production process of the present invention is useful as a production process for retinol on an industrial scale, because the starting material, diol compound (2), can easily be derived from relatively inexpensive starting materials, linalool or geraniol, without taking a route through C₁₃ β-ionone or C₁₀ aldehyde derivatives.

## Claims

1. A halogenated sulfone derivative of general formula (1): 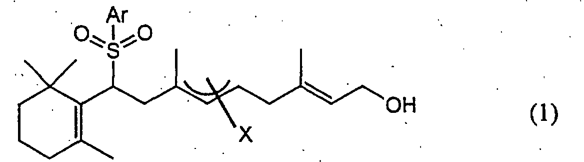 wherein Ar is optionally substituted aryl and X is halogen.

2. A production process for a halogenated sulfone derivative of general formula (1) as set forth in claim 1, characterized in that a diol compound of general formula (2): 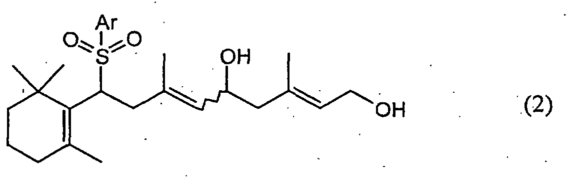 wherein Ar is optionally substituted aryl, is reacted with a Group IV transition metal halide.

3. The production process for retinol, characterized in that a halogenated sulfone derivative of general formula (1) as set forth in claim 1 is reacted with a base.

4. The production process for retinol, characterized in that a diol compound of general formula (2) as set forth in claim 2 is reacted with a Group IV transition metal halide to give a halogenated sulfone derivative of general formula (1) as set forth in claim 1 and the resulting halogenated sulfone derivative is then reacted with a base.

5. The production process according to claim 2 or 4, wherein the Group IV transition metal is titanium.

6. The production process according to claim 2 or 4, wherein the Group IV transition metal halide is a chloride of titanium.

7. The production process according to claim 6, wherein the chloride of titanium is titanium tetrachloride, dichlorotitanium diisopropoxide or chlorotitanium tiriisopropoxide.

8. The production process according to claim 2 or 4, characterized in that the reaction of a diol compound of general formula (2) as set forth in claim 2 with a Group IV transition metal halide involves the presence of an ether compound.

9. The production process according to claim 8, wherein the ether compound is dimethoxyethane or tetrahydrofuran.

10. The production process according to claim 3 or 4, wherein the base is an alkali metal compound or an alkaline earth metal compound.

11. The production process according to claim 10, wherein the alkali metal compound is an alkali metal hydroxide or an alkali metal alkoxide.
